# EUROPEAN PATENT SPECIFICATION

(11) **EP 3 326 582 B1**
(45) Date of publication and mention of the grant of the patent: **16.09.2020**
(21) Application number: 17275186.9
(22) Date of filing: 23.11.2017
(51) Int. Cl.: A61F 2/07, A61F 2/90, A61F 2/06, A61F 2/89

(54) **STENT GRAFT WITH TOP MESH STENT**
STENTGRAFT MIT EINEM OBEREN MESH-STENT
ENDOPROTHÈSE AVEC STENT À MAILLES SUPÉRIEUR

(30) Priority: 29.11.2016 US 201662427621 P
(43) Date of publication of application: 30.05.2018
(73) Proprietor: Cook Medical Technologies LLC, Bloomington, IN 47404 (US)
(72) Inventor: Bradway, Ryan, West Lafayette, IN 47906 (US)
(74) Representative: Williams Powell

(56) References cited:
- WO-A1-2012/088475
- WO-A2-2007/079153
- US-A1- 2009 270 965
- US-A1- 2010 022 940
- US-A1- 2011 118 816

## Description

The present disclosure relates generally to medical devices, and in embodiments to fenestrated prostheses for implantation within a human or animal body for repair of damaged vessels, ducts, or other physiological pathways.

### Background

US 2009/0270965 discloses an endovascular prosthesis for ascending aorta.

Endovascular methods have been proposed for treatment of diseases of the aorta such as aortic dissection and aortic aneurysm. Using prostheses, such as stent grafts, to treat aneurysms is common in the medical field. Stent grafts are deployed by accessing a vasculature with a small incision in the skin and guiding a delivery system to the target area. This endoluminal delivery is less invasive and generally preferred over more intrusive forms of surgery. Multiple stent grafts may be implanted using endoluminal delivery to provide a system of interconnected stent grafts. Interconnected stent grafts may be made of fenestrated stent grafts and smaller side branch grafts, including bifurcated components.

Such methods have been proposed particularly when the diseased portion of the aorta is along the ascending aorta, the sinotubular junction (STJ), and into the aortic root. When the diseased portion of the aorta is located in such area, endovascular techniques for treating these diseases are somewhat more difficult because of variable and non-circular profile of the STJ and aorta root, the presence of major arteries in the region, and the proximity to the heart.

For instance, for treatment of aortic aneurysms and/or dissections in particular, it is necessary to introduce the stent graft through the aorta system and in a region of the ascending aorta where there may be strong blood flow. Furthermore, in the ascending aorta and STJ region there are major branch vessels extending therefrom, such as the first and left coronary arteries. During and/or after treatment of an aneurysm or dissection in this vessel region of the aorta, it is desirable for blood supply to continue to flow to these branch arteries. For this purpose, stent grafts are configured, typically with fenestrations or side branches, to the flow.

### SUMMARY

Aspects of the present invention seek to provide improved devices for example for implantation in this vessel region.

According to an aspect of the invention, there is provided a stent graft as in claim 1.

In an example, a stent graft having a radially compressed configuration and a radially expanded configuration is disclosed. The stent graft includes a tubular graft body having a first end opening and a second end opening. A stent structure is coupled along the graft body, and an end of the stent structure that is in closest proximity to the first end opening of the graft body is spaced from the first end opening to define a clearance region of the graft body. An annular mesh top stent includes a first portion coupled along an inner surface of the graft body along the clearance region below the first end opening at attachment locations, and a second portion extending beyond the first end opening outside of the graft body. During radial expansion, the first portion is retracted to a retracted longitudinal length, and the second portion expands to a bulbous shape having a greater cross-sectional area than the first portion. The attachment locations are circumferentially spaced from one another at non-axially adjacent locations, whereby tearing of the graft body is inhibited during retraction of the annular mesh top stent.

In another example, a stent includes a tubular graft body disposed along a longitudinal axis, with the tubular graft body having an inflow end opening and an outflow end opening. The stent includes a plurality of discrete self-expanding stents coupled along a wall of the graft body and axially spaced from one another. The stent includes a self-expanding annular mesh stent having an inner portion coupled along an inner surface of the graft body at a series of suture attachments arranged in a single annular row, and a projecting portion extending beyond the inflow end opening of the graft body. In the radially compressed configuration, the inner portion has an extended longitudinal length. In the radially expanded configuration, the inner portion is retracted to a retracted longitudinal length, and a cross-sectional shape of the second portion is a three-sided shape. One of the discrete self-expanding stents that is in closest proximity to the inflow end opening of the graft body is spaced from the inflow end opening by a clearance length sized longer than the extended length.

In another example, a method of deploying a stent graft is disclosed. The stent graft includes a tubular graft body having a graft wall defining a lumen extending between a first end opening and a second end opening. A stent structure is coupled along the graft wall. An annular mesh top stent extends between a first end and a second end. The annular mesh top stent includes a first portion coupled along an inner surface of the graft body below the first end opening at attachment locations, and a second portion extending beyond the first end opening outside of the graft body. The attachment locations are circumferentially spaced from one another in an annular pattern, and an end of the stent structure that is in closest proximity to the first end opening of the graft body is spaced from the first end opening by a clearance length. The method includes one or more of the following steps. A step includes positioning the stent graft in a radially compressed configuration within a vessel of a patient. The first potion has an extended length that is less than the clearance length. A step includes radially expanding the stent graft so that the first portion retracts to a retracted length, and a bulbous portion of the second portion has a greater cross-sectional area and a different cross-sectional shape than the first portion.

### BRIEF DESCRIPTION OF THE DRAWINGS

Embodiments of the invention may be better understood with reference to the following drawings and description, which are provided by way of example only. The components in the figures are not necessarily to scale, emphasis instead being placed upon illustrating the principles of embodiments of the invention. Moreover, in the figures, like referenced numerals designate corresponding parts throughout the different views.
FIG. 1 is a perspective view of an example of a stent graft.
FIG. 2 is a side view of the stent graft depicted in FIG. 1.
FIGS. 3-6 are cross-sectional views of the stent graft depicted in FIG. 2 taken along lines 3-6, respectively.
FIG. 7 is a perspective view of another example of a stent graft.
FIGS. 8-9 depict the radial movement of the stent graft between a radially expanded configuration and a radially compressed configuration.
FIG. 10 depicts an example stent graft loaded on a delivery device.
FIGS. 11-12 depict delivery of the stent graft with the delivery device of FIG. 10 within a body vessel of a patient.
FIGS. 13-14 are end views of examples of mandrels used to form a top stent.

### DETAILED DESCRIPTION OF THE PREFERRED EMBODIMENTS

Stent grafts are disclosed for implantation within a human or animal body for repair of damaged vessels, ducts, or other physiological pathways. In a particular example, the stent graft may be implanted for treatment of diseases of the aorta such as aortic dissection and aortic aneurysm, and specifically, the ascending aorta, STJ region and aorta root. The body of the graft may be implanted into a primary vessel, and a top mesh stent extending from the graft body may be at least partially uncovered to provide access and blood perfusion to a branch artery, such as, for example, the right and left coronary arteries, extending from the primary vessel. The top mesh stent is shaped to span the cross-sectional shape of the part of the vessel in which it is implanted. The stent grafts described herein are configured to provide improved positioning and transitioning between a circular primary vessel and a noncircular vessel and achieve sealing along such transition.

In the present application, the term "proximal" when referring to a delivery device refers to a direction that is farthest away from an operator using a delivery device, while the term "distal" refers to a direction that is generally closest to the operator using the delivery device. The proximal and distal ends of a delivery device may also be referred to as an introduction end of the delivery device and an operator end of the delivery device, respectively. The term "operator end" of the delivery device is that portion of the device that is intended to remain outside of a patient during a procedure. The term "introduction end" of the delivery device, which is opposite to the operator end, is that portion of the device that is intended to be inserted within a patient during a procedure. When referring to the prosthesis itself relative to the delivery device, the proximal end of the prosthesis is that part of the prosthesis closest in proximity to the introduction end of the delivery device and the distal end of the prosthesis is that end that is closest in proximity to the operator end of the delivery device. When referring to the prosthesis relative to placement in the human body of the patient, the ends of the various devices and parts of devices may be referred to as the inflow end (that end that receives fluid first, and the outflow end (that end from which the fluid exits).

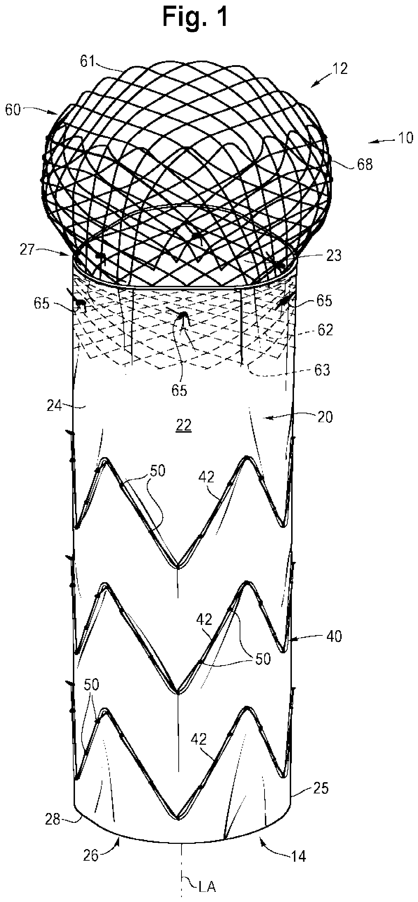

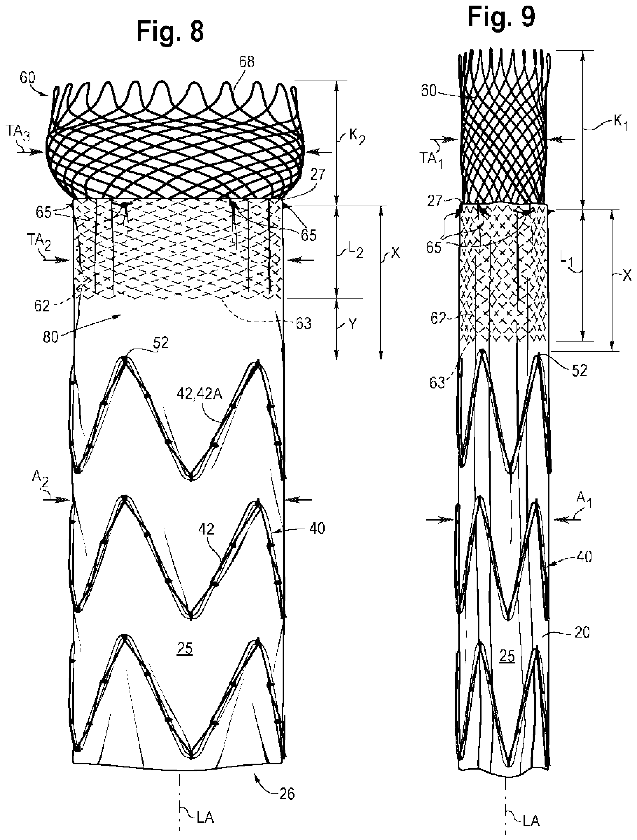

**FIG. 1** depicts one example of a stent graft 10 having a proximal, inflow end 12 and a distal, outflow end 14. The stent graft 10 includes a graft body 20 comprising a graft material 22 shaped in a manner to define an inner surface 23 and an outer surface 24 of the graft body 20. The inner surface 23 and the outer surface 24 define the wall thickness of the graft body wall 25. The graft body 20 may be configured as a generally tubular member having a substantially cylindrical shape, although, in other examples, one or more axial regions of the graft body 20 may be tapered. The inner surface 23 of the graft body 20 defines a main lumen 26 extending longitudinally about an axis LA within the graft body 20 between a proximal first end opening 27 and a distal second end opening 28 thereof. The proximal first end opening 27 is opposite to the distal second end opening 28 and in closer proximity to the inflow end 12. The second end opening 28 may coincide with the outflow end 14 of the stent graft 10. The main lumen 26 may be suitable for passing a fluid, such as, for example, blood or other body fluid therethrough. The stent graft 10 is movable between a radially compressed configuration (shown in **FIG. 9**) and a radially expanded configuration (shown in **FIGS. 1****,** **2** **and** **7****-8**).

The term "graft body" describes an object, device, or structure that is joined or that is capable of being joined to a body part to enhance, repair, or replace a portion or a function of that body part. Graft body 20 may be used to repair body vessels include, for example, films, coatings, or sheets of graft material that are formed or adapted to conform to the body vessel that is being enhanced, repaired, or replaced. The graft material 22 may be formed from a biocompatible material that is substantially non-toxic in the in vivo environment of its intended use and substantially unrejected by the patient's physiological system (that is, is non-antigenic). For example, the graft material 22 may be made of an expanded polytetrafluoroethylene (ePTFE), polytetrafluoroethylene (PTFE), silicone, polyurethane, polyamide (nylon), polyethylene, polypropylene, polyaramids, polyacrylonitrile, cellulose, or another flexible biocompatible material. The graft material 22 also may be made of known fabric graft materials, for example, woven polyester such as DACRON® from Invista (Wichita, Kans.), polyetherurethanes such as THORALON® from Thoratec Corporation (Pleasanton, Calif.), or polyethylene such as an ultra-high molecular weight polyethylene (UHMwPE) such as DYNEEMA® from DSM Dyneema LLC (Stanley, N.C.). Additionally, or alternatively, materials that are not inherently biocompatible may be subjected to surface modifications to render the materials biocompatible. Examples of surface modifications may include, for example, graft polymerization of biocompatible polymers on the surface, coating of the surface with a crosslinked biocompatible polymer, chemical modification with biocompatible functional groups, or immobilization of a compatibilizing agent such as heparin or other biocompatible substances. Thus, any fibrous material having sufficient strength to survive in the in vivo environment may be used to form a textile graft, provided the final textile is biocompatible.

The graft material 22 may include a bioremodelable material such as reconstituted or naturally-derived collagenous materials, extracellular matrix (ECM) material, submucosa, renal capsule membrane, dermal collagen, dura mater, pericardium, fascia lata, serosa, peritoneum or basement membrane layers, or intestinal submucosa, including small intestinal submucosa (SIS), stomach submucosa, urinary bladder submucosa, or uterine submucosa. One non-limiting example of a suitable remodelable material is SURGISIS® BIODESIGN™ from Cook Medical (Bloomington, Ind.). Another suitable remodelable material is the graft prosthesis material described in U.S. Pat. No. 6,206,931 to Cook et al. Additionally, or alternatively, the graft material 22 may be made of any of the materials described in U.S. Pat. No. 7,407,509 to Greenberg et al. or U.S. Patent Application Pub. No. 2009/0171451 by Kuppurathanam et al.

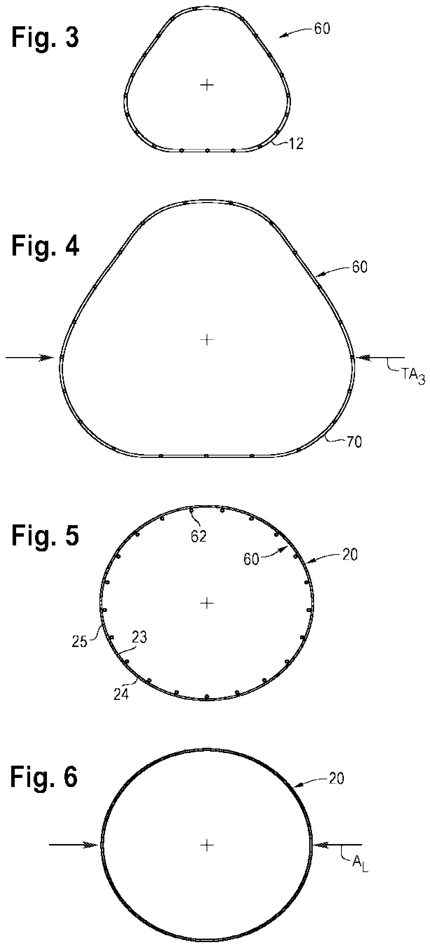

An expandable stent structure 40 is coupled along the graft body wall 25 of the graft body 20, for example, via suture attachment ties 50. The stent structure 40 may move to the radially expanded configuration (see **FIG. 8**) from a smaller, radially compressed configuration (**FIG. 9**). The stent structure 40 coupled to the graft body 20, when radially expanded and implanted, is configured to exert a radially outward force on an interior wall of the body vessel. The force may be suitable to provide support to the body vessel at the point of treatment and maintain the main lumen 26 of the graft body 20 open up to its full cross-sectional area, shown as a second graft cross-sectional area A2 in **FIG. 6****.** The stent structure 40 may be a single unitary structure or may comprise of a plurality of discrete stents.

In one example, the stent structure 40 may comprise of a plurality of discrete stents 42 axially spaced from one another and coupled along the inner surface 23, the outer surface 24, or both of the graft body wall 25. As shown in **FIGS. 8-9****,** the proximal end 52 of the stent structure 40 closest in proximity to the proximal first end opening 27 may be spaced from the proximal first end opening 27 by a clearance length X or defining a clearance region of the graft body as described below. When the stent structure 40 is a discrete stent 42, as shown in the figures, the proximal end 52 is the proximal end of the proximal most stent 42A of the discrete stents 42 that is in closest proximity to the proximal first end opening 27 than the others.

The stents 42 depicted are shown being defined by a plurality of interconnected unit stent members arranged in an undulating pattern, such as, for example, a zigzag or serpentine pattern. The term "stent" means any device or structure that provides or is configured to provide rigidity, expansion force, or support to a body part, for example, a diseased, damaged, or otherwise compromised body lumen. The stents 42 may include any suitable biocompatible material, including, but not limited to, fabrics, metals, plastics, and the like. Examples of suitable materials include metals such as stainless steel and nitinol, and plastics such as polyethylene terephthalate ("PET"), polytetrafluoroethylene ("PTFE") and polyurethane. The stents 42 may be "expandable," that is, it may be capable of being expanded to a larger-dimension configuration. The stents 42 may expand by virtue of its own resilience (that is, self-expanding), upon the application of an external force (that is, balloon-expandable), or by a combination of both. In one example, the stents 42 may have one or more self-expanding portions and one or more balloon-expandable portions. In one example, the stents 42 shown are self-expanding under their inherent resilience. An example of a suitable self-expanding stent includes Z-STENTS™, which are available from Cook Inc., Bloomington, Ind., USA.

Returning to **FIG. 1****,** the stent graft 10 also includes a top stent 60 shown projecting from the proximal first end opening 27 of the graft body 20. The top stent 60 is comprised of a stent pattern defined in an annular shape extending from a proximal first end 61 that may coincide with the inflow end 12 of the stent graft 10, and a distal second end 63 at the opposite end. In one example, the top stent 60 has a different stent construction than the stent structure 40. In the example shown, the top stent 60 has a wire mesh construction generally with more cells (lower cell opening-to-stent material ratio than that of the stent structure 40), and the stent structure 40 comprises a series of zigzag discrete stents generally with less cells (higher cell opening-to-stent material ratio). As used herein, the term "mesh" is used to refer generally to stents constructed braided, woven, braiding, knitted, crocheted, or sutured stent wire material.

The density of the cells should be selected to allow the top stent 60 to protrude over branching vessels without detrimentally blocking blood flow or increasing clotting risks and to conform to and support the local anatomy in which is implanted. The number of cells about the circumference of the top stent 60 may depend on several factors, including the cell size, the cross-sectional area of the top stent, and spacing between the cells. It may be appreciated that any number of cells may be provided. The cells may be sufficiently spaced to result in a cell opening-to-stent material ratio of at least 1:1 (about 50% open space and 50% stent material). In other examples, the ratio may fall within the range between approximately 1.5:1 (about 60% open space and 40% stent material) and 4:1 (about 80% open space and 20% stent material). The stent material or strut width associated with the spacing between adjacent cells may fall within the range of 0.05 mm and about 0.3 mm. The top stent 60 may include closed cell and/or open cell configurations. The shape of the cells may include, such as but not limited to, diamond as shown, hexagon or double hexagon, auxetic, hourglass, octagon, or any combination of such shapes to define hybrid configurations.

A first inner portion 62 of the top stent 60 may be disposed within the lumen 26 of the stent graft 10. The first inner portion 62 may be annular segment coupled along the inner surface 23 of the graft body wall 25 below the proximal first end opening 27 at suture attachment locations 65. A second projecting portion 68 of the top stent 60, contiguous with the first inner portion 62, is disposed outside the lumen 26 of the graft body 20 to extend beyond the proximal first end opening 27. The top stent 60 may be self-expanding under its inherent resilience or may be heat activated wherein the top stent 60 self-expands upon reaching a predetermined temperature or range of temperatures when made from a shape memory metal alloy.

The first inner and second projecting portions 62, 68, when expanded, may have a cross-sectional shape, including but not limited to, a rectangular cross-section, oval cross-section, triangular cross-section, an irregular (or non-uniform) cross-section, or some combination thereof. The first inner and second projecting portions 62, 68, when expanded, are shown having different cross-sectional shapes. For example, the first inner portion 62, when expanded, may have a circular cross-sectional shape corresponding to the expanded cross-sectional shape of the graft body 20, as shown in **FIG. 5****.** Further, the second projecting portion 68, when expanded, may have a non-circular cross-sectional shape, such as, for example, three-sided cross-section (shown in **FIGS. 3-4**) or irregular (or non-uniform) cross-section.

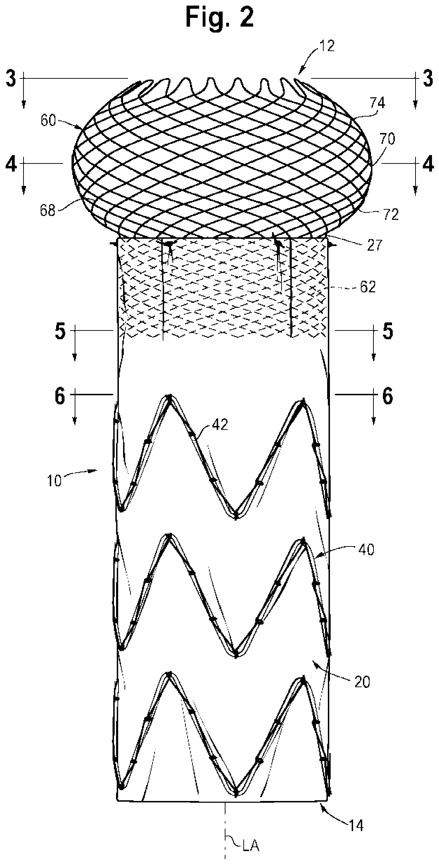

**FIG. 2** depicts one example profile of the top stent 60. Here, the first projecting portion 68 of the top stent 60, when radially expanded, includes a bulbous intermediate portion 70 and an outwardly tapered portion 72 longitudinally extending outward from the proximal first end opening 27 of the graft body 20 to the bulbous intermediate portion 70. The outwardly tapered portion 72 includes an increasingly larger cross-sectional area along the longitudinal axis LA in the proximal direction. Further, the first projecting portion 68 of the top stent 60, when radially expanded, may include an inwardly tapered portion 74 including a decreasingly smaller cross-sectional area to the inflow end 12 of the stent graft 10 along the longitudinal axis LA in the proximal direction. The tapering of tapered portions 72, 74 may have any suitable profile to match the anatomy in which it is implanted, such as for example, a rounded profile and/or a linear profile.

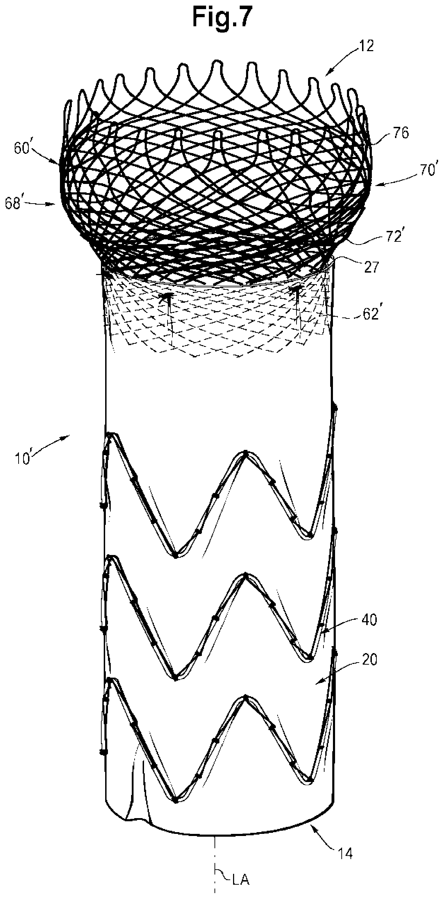

**FIG. 7** depicts another example of the stent graft (referred to as stent graft 10'), with another example of the top stent 60' with a different profile. Here, the first projecting portion 68' of the top stent 60, when radially expanded, includes the bulbous intermediate portion 70' and the outwardly tapered portion 72' extending outward from the proximal first end opening 27 of the graft body 20 to the bulbous intermediate portion 70'. The outwardly tapered portion 72' includes an increasingly larger cross-sectional area along the longitudinal axis LA in the proximal direction. Further, the first projecting portion 68' of the top stent 60', when radially expanded, includes a cylindrical portion 76' having a uniform cross-sectional area to the inflow end 12 of the stent graft 10' along the longitudinal axis LA in the proximal direction.

The following description will be applicable to the stent grafts 10, 10', although the stent graft 10 will be used as the exemplary stent graft. In **FIG. 9****,** when the stent graft 10 is in the radially compressed configuration, the stent structure 40 coupled to the graft body 20 defines a first graft cross-sectional area A1 and a first graft cross-sectional shape. Further, the first inner and second projecting portions 62, 68 of the top stent 60 have generally a first top stent cross-sectional area TA1 and first cross-sectional shape. Here, the shape and cross-sectional area of each may be defined generally by the size and inner lumen shape, in most instances circular, of an outer sheath disposed over the stent graft during delivery. The first graft cross-sectional area A1 and the first top stent cross-sectional area TA1 and respective cross-sectional shapes may be substantially the same, within the degree of the added wall thickness (for example, 0.004 mm to 1 mm) of the graft body 20.

In **FIG. 8****,** the stent graft 10 is expanded from the radially compressed configuration, and the stent structure 40 coupled to the graft body 20 expands to a second graft cross-sectional area A2 that is larger than the first graft cross-sectional area A1. Further, the first inner portion 62 of the top stent 60 expands to a second top stent cross-sectional area TA2 that is larger than the first top stent cross-sectional area TA1. The second top stent and second graft cross-sectional areas TA2, A2 and respective cross-sectional shapes may be substantially the same, in most instances rounded or circular, within the degree of the added wall thickness of the graft body. Further, the second projecting portion 68 of the top stent 60 expands to a cross-sectional area larger than the first top stent cross-sectional area TA1. For example, at least a portion of the second projecting portion 68 of the top stent 60 (for example, the bulbous intermediate portion 70) expands to a third top stent cross-sectional area TA3 that is larger than the first top stent cross-sectional area TA1. The third top stent cross-sectional area TA3 is also larger than the second top stent and second graft cross-sectional areas TA2, A2. In one example, the first diameter corresponding to the second top stent cross-sectional area TA2 is a diameter in the range of 22-36 mm, and the second diameter corresponding to the third top stent cross-sectional area is a diameter in the range of 29-45 mm. To this end, the ratio between the second diameter and the first diameter is between 1.25 and 1.35. In one example, a portion of the second projecting portion 68 has a greater cross-sectional area and a different cross-sectional shape than that of the graft body 20, when radially expanded.

Also shown in **FIGS. 8-9****,** the length of the top stent 60 shortens when expanding from the radially compressed configuration to the radially expanded configuration. In **FIG. 9****,** the first inner potion 62 has an extended, first length L1 and the second projecting portion 68 has a first projecting length K1, when the stent graft is in radially compressed configuration. Upon expansion, in **FIG. 8****,** the first inner potion 62 retracts to a retracted second length L2 that is less than the first length L1, and the second projecting portion 68 retracts to a second projecting retracted length K2 that is less than the first projecting length K1. The retracted second length L2 of the first inner portion 62 is suitable to provide a sealing zone along the outer graft body 20 where the first inner portion 62 is a liner. The length difference between the clearance length X and the second retracted length L2 may define an unstented region or length Y along the graft body where any stent structure is omitted, as shown in **FIG. 6****.** The unstented length Y may be sufficiently long to prevent overlapping of the first inner portion 62 and the proximalmost stent 42A during curvature of the stent graft 10. In other words, the retracted second length L2 should permit enough spacing between the first inner portion 62 and the stent 42A, such that when the stent graft is curved the first inner portion 62 and the stent 42A are disposed adjacent to one another in a non-overlapping relationship. It is noted that the clearance length X is at least longer than the first length L1. The total elongated length of the top stent 60 would be the sum of the first projecting length K1 and the first length L1. The total retracted length of the top stent 60 would be the sum of the second projecting length K2 and the second length L2. The ratio of the total elongated length to the total retracted length of the top stent may be, for example but not limited to, at least 1.5. In other examples, the ratio of the first length L1 to the second length L2 of the first inner portion 62 of the top stent 60 may be, for example but not limited to, at least 1.5.

Adjacent stents may be spaced a suitable distance from one another. The spacing between the adjacent stents may be sufficiently large to enable such flexibility of the graft body. In other words, an unsupported longitudinal section of the graft material of the graft body 20 between the adjacent stents may be sufficiently flexible to enable movement of the graft body. The stent sizing, spacing, and design may be determined so that there is no stent-to-stent contact even in tortuous anatomy. Stents preferably may be placed to maximize prosthesis flexibility while maintaining patency, as well as reducing material wear and stent fatigue.

In the example shown, with reference to **FIG. 6****,** the second graft cross-sectional area A2 of the graft body 20 has a substantially cylindrical shape when the stent graft 10 is in the radially expanded configuration. Referring to **FIG. 5****,** the second top stent cross-sectional area TA2 of the first inner portion 62 may be substantially cylindrical shape and concentric with the expanded graft body 20. In the example shown, the second projecting cross-sectional shape of the second projecting portion 68 has a non-cylindrical or non-circular shape, and the third top stent cross-sectional area TA3 of the second projecting portion 68 is greater than the second graft cross-sectional area A2. In one example, the second projecting cross-sectional shape of the second projecting portion is three-sided (that is, triangular or tri-lobal shape), as shown in **FIGS. 3-4****.**

The suture attachment locations 65 are circumferentially spaced from one another in non-axial adjacent locations. For example, the number of suture attachment locations 65 may include two, three, four, five, six or more. In one example, the suture attachment locations 65 shown are arranged in a single row of suture attachments in an annular arrangement. In another example, the circumferentially adjacent suture attachment may be axially offset to small degree to define an annular zigzag pattern. The suture attachment locations 65 in these annular arrangements may allow free movement between the extension and retraction of the first inner portion 62 of the top stent 60 along the graft body wall 25 without comprising the graft material. Placing additional suture attachments axially adjacent to and in substantial axial alignment to the suture attachment locations 65 or in a direct side by side longitudinal arrangement along the graft body 20 may leave the graft body 20 susceptible to tearing during the axial movement of the first inner portion 62, which may result in leak paths.

The clearance length X may provide one of several benefits. As shown in **FIG. 8****,** the clearance length X may provide an annular external segment 80 along the outer surface 24 of the graft body 20 for improved sealing against the body vessel wall. To this end, the radial expansion of the first inner portion 62 coupled along the inner surface 23 of the graft body 20 places the annular external segment 80 of graft material 22 apposed to the body vessel wall. In one example, the annual external segment 80 remains unobstructed from any portion of the stent structure 40. The clearance length X may also provide clearance for the free movement of the extension and retraction of the first inner portion 62 of the top stent 60 along the graft body wall 25. Engagement between the axially movable first inner portion 62 and the stent structure 40, shown as the proximalmost stent 42A, may leave the graft body 20 susceptible to tearing when compressed and increase the cross-sectional profile of the stent graft. The clearance length X may also provide clearance between the first inner portion 62 of the top stent 60 and the stent structure 40 when the stent graft 10 is longitudinally bent or curved either during delivery or after implantation.

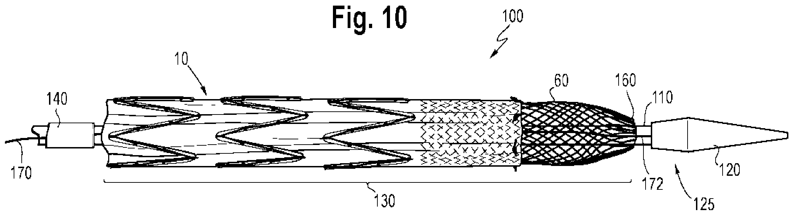

One common procedure for implanting a stent graft (the example of the stent grafts described herein) may involve at least a two-step process. First, if necessary, the diseased vessel may be dilated with a balloon or other dilatation device prior to stent graft delivery. Next, the stent graft may be delivered. In **FIG. 10****,** a delivery device 100 is shown including an inner cannula 110 and a nosecone dilator 120 at a proximal, introduction end 125 of the delivery device 100 and coupled to the inner cannula 110. The stent graft 10 may be loaded on a proximal region 130 of the inner cannula 110 and restrained in the radially compressed configuration within an outer sheath 140 for delivery to the targeted vessel. The tip end 160, or portion adjacent thereto, of the top stent 60 may be further radially restrained by a trigger wire 170 as shown. The proximal end 172 of the trigger wire 170 may be woven into open cells of the top stent 60. When the trigger wire 170 is secured in tension, for example, at its distal end proximate the operator's end of the delivery device, the tip end 160 of the top stent 60 is gathered closer the inner cannula 110 than would be otherwise obtainable without the trigger wire 170.

The delivery systems described herein may need various other components in order to obtain a delivery and deployment system that is optimally suited for its intended purpose. These include and are not limited to various outer sheaths, pushers, trigger wires, stoppers, guide wires, and the like. For example, the Zenith® Thoracic Aortic Aneurysm Endovascular Graft uses a delivery system that is commercially available from Cook Inc., Bloomington, Indiana, and may be suitable for delivering and deploying an aortic prosthesis in accordance with the present embodiments.

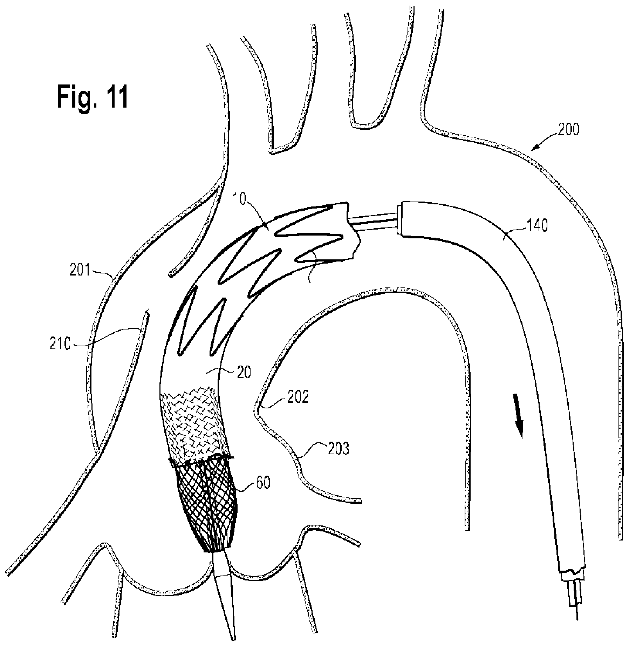

In **FIG. 11****,** the stent graft 10 may then be guided with a primary vessel 200 to the target anatomy via the delivery device 100, with the outer sheath 140 in the extended position (not shown) to restrain the stent graft 10 in the radially compressed configuration. In the example shown, the primary vessel 200 is shown as the ascending aorta 201, STJ region 202 and aorta root 203 at the sinus of Valsalva, and the treatment site 210 is an aortic dissection of this region. The delivery device 100 is guided over a guide wire (not shown) inserted within a longitudinal lumen of the inner cannula 110. At least a portion of the nosecone dilator 120 may be inserted carefully through the aorta root as shown in order to appropriately place the stent graft. The inner cannula 110 and the nosecone dilator 120 may be held in place, while the physician operator applies a distal withdrawing force to the outer sheath 140 in the distal direction (shown by the arrow) to remove it from the loaded stent graft 10. The outer sheath 140 may be released partially from the stent graft 10, keeping the outflow end 14 restrained within the outer sheath 140 to provide opportunities to manipulate and orient the stent graft 10 relative to the treatment site.

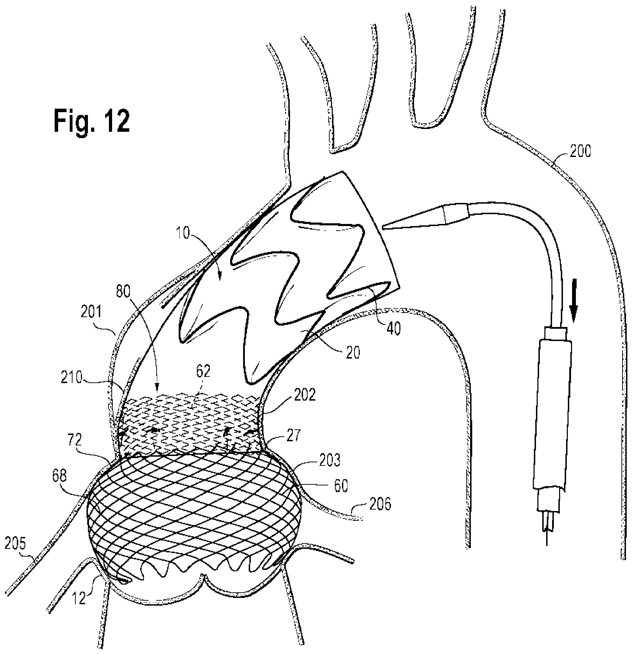

Once at least partially released from the outer sheath 140, the stent structure 40 of the stent graft 10 may radially expand under its own resilience until the graft body contacts and presses against the treatment site 210 at the vessel wall of the primary vessel, shown as the ascending aorta 201, as shown in **FIG. 12****.** With the delivery device 100 with the trigger wire 170, as shown, the physician operator applies a distal withdrawing force to the trigger wire 170 to remove it from the top stent 60 to allow the top stent 60 to radially expand. For example, the top stent upon reaching a desired temperature from the patient's body temperature may expand to its radially expanded configuration. In one example, the stent graft 10 is placed such that the proximal first end opening 27 of the graft body 20 terminates proximate the STJ junction such that the second projection portion 68 of the top stent 60 conforms to the surrounding anatomy, shown as the aorta root 203, while maintaining a distance from the branch vessels, shown as the coronary arteries 205, 206, so as to not occlude the branch vessels. The inflow end 12 of the stent graft 10 should be placed short of the valve leaflets of the aortic valve so as to minimize interference with their native function. The second projection portion 68 of the top stent 60, under radial expansion, is shown shaped to conform to size and cross-sectional shape of the aorta root 203, which may be described as tri-lobal.

As may be seen, the radial expansion of the first inner portion 62 places the annular segment 80 of graft material 22 apposed to the vessel wall of the ascending aorta 201 to form a seal between the graft body and the vessel wall. The radial expansion of the first inner portion 62 places the annular segment 80 of graft material 22 apposed to the vessel wall at the STJ region 202 to form a seal between the graft body and the STJ vessel wall. **FIG. 12** shows the unstented length between the first inner portion 62 and the proximalmost stent of the stent structure 40 is sufficient to prevent overlapping of the first inner portion 62 and the proximalmost stent during curvature of the stent graft 10. This nonoverlapping arrangement may improve sealing between the graft body 20 apposed to the STJ region vessel wall since the proximalmost stent of the stent structure is removed from this region. Further, the radial expansion of the second projecting portion 68 that is greater than the graft body 20 allows the second projection portion 68 to conform to the size and shape of the aorta root that has a bulbous shape. The flaring of the top stent 60 at the outwardly tapered portion 72 extending outward from the proximal first end opening 27 of the graft body 20 to the bulbous intermediate portion forms a physical flange at the STJ to inhibit downstream migration of the stent graft 10 away from the aorta root due to the blood flow and pressure.

In some procedures, the stent graft 10 may be delivered with the assistance of an endoscope and/or a fluoroscope. An endoscope provides visualization of the lumen as well as working channels through which devices and instruments may be delivered to the site of implantation. A fluoroscope also provides visualization of the patient anatomy to aid in placement of an implantable device, particularly in the aortic system. After implantation, the delivery device 100, as shown in **FIG. 12****,** and any other devices (for example, wire guides, catheters, etc.) may be removed.

Top stents 60, 60' may be formed by any suitable method as is known in the art. For example, the top stent may be laser cut from a cannula made of a shape memory metal alloy in the pattern. In one example, the top stents may be fabricated by braiding, weaving, knitting, crocheting, welding, suturing, or otherwise machining together one or more filaments or wires into a tubular structure. As known in the art, one or more mandrels and restraining devices may be used for producing the top stent. For example, a mandrel having a cross-sectional area and shape corresponding to the final cross-sectional area and shape of the top stent may be provided. The mandrel may have specifically designed features (for example, pinholes, pins, grooves and detents) for creating such a top stent design. A variety of braiding patterns are possible, such as for example, one-under and one-over patterns or two-under and two-over patterns. An end of the mandrel includes a series of pinholes disposed around the circumference of the mandrel. Pins may be located in the pinholes and project outward beyond the mandrel surface. The mandrels, restraining devices, and pins are made of materials, such as but not limited to brass, copper, or stainless steel, suitable to withstand thermal curing temperatures of the metal wire.

A single metal filament or wire or plurality of metal filaments or wires may be wound and/or braided in a helical pattern about the mandrel forming a cylindrical tubular shape. The metal filaments or wires may be various cross-sectional shapes. For example, the filaments or wires may be flat in shape (or rectangular or oval cross section) or may have a circular cross-section. The metal filaments or wires may have any suitable initial diameter, such as for example, from about 0.1 to about 0.3 mm. Metal filaments or wires may be bent around the pins, resulting in the formation of bends formed at the inflow end of 61 the top stent 60. A restraining sleeve or wire loop may extend around the filaments or wires beneath the pins and along the mandrel surface to help restrain them from returning to their original configuration. At least some of the bends may be formed into loops by twisting the bends. Other embodiments of top stents may be contemplated, including but not limited to, those produced from two or more metal filaments or wires having helical or other configurations. The metal filament or wire is notably made of shape memory metal alloy, such as nitinol, or other alloy/material with similar characteristics, including but not limited to FeMnSi, FeNiCo(Al,Ti)Ta (with or without B), or Fe-Pt or Fe-Pd alloys.

After winding, weaving or braiding the metal filaments or wires is complete, the metal filament or wire ends may be fixed or coupled together, for example but not limited to, by weldments, crimped metal clips, twists and/or tying, to form a complete stent structure and in a manner to avoid inadvertent uncoupling during production or when implanted. The wound stent structure may be heated to a suitable temperature and period to time, such as, for example, but not limited to, about 500° C. for up to, such as, for example, about 120 minutes, and then allowed to cool to room temperature where the metal wires may be configured to exhibit superelastic properties. Deformation of the shape of the top stent structure with superelasticity may occur by applying a restraining force thereto. As a result, the shape memory of the top stent may be activated by removing the restraining force, thereby allowing the top stent to recover its initial cross-sectional area and shape, or within some degree of the area and shape, as appreciated by one of ordinary skill in the art.

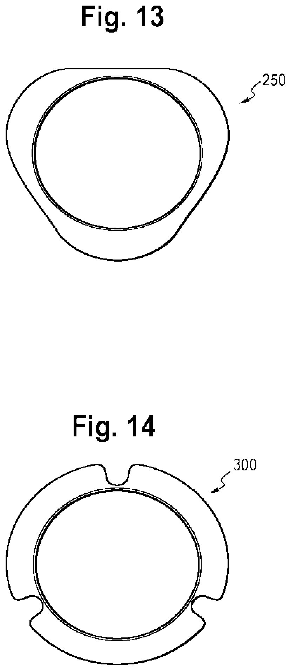

The processing of the metal filament or wire may be such that when the restraining force is removed, the top stent may remain deformed until being activated by heating the top stent to a desired temperature, such as the patient's body temperature, to recover its initial cross-sectional area and shape. In one example, the mandrel is shaped and sized similar to the final shape and size of the top stent. In other words, the mandrel has a first portion shaped and sized similar to the final shape and size of the first inner portion, and a second portion shaped and sized similar to the final shape and size of the second projecting portion. **FIG. 13** shows an end view of an example mandrel 250 having a three-sided shape to form an aspect of the profile of the second projection portion of the top stent. **FIG. 14** shows an end view of another example of a slotted mandrel 300 having a circular shape to form an aspect of the profile of the first inner portion of the top stent. Note, the relative sizes and shapes of the mandrels are for illustrative purposes and may not reflect their actual size.

Additional process and appropriately shaped mandrels may be used to make the final shape and cross-sectional area of the first inner portions and the second projecting portions of the top stent. The top stent structure, after the initial processing with the mandrel and heating, may be secured to a second mandrel by a restraining sleeve or wire loops along the stent structure to restrain the stent structure against the mandrel surface. The stent structure is reheated using a similar temperature and period of time as the first heating. After cooling down to room temperature, the metal wires may exhibit superelastic properties. In one example, the initial mandrel may be sized and shaped similar to the size and shape of the first inner portion. A portion of the stent structure may be reconfigured to wrap around and be restrained to the larger second mandrel sized and shaped similarly to the second projecting portion. In another example, the initial mandrel may be sized similar to the size of the second projecting portion. A portion of the stent structure may be reconfigured to wrap around and be restrained to the smaller second mandrel sized and shaped similarly to the first inner portion. Another portion of the stent structure may be reconfigured to wrap around and be restrained to a third mandrel shaped similarly to the second projecting portion.

While various embodiments of the invention have been described, the invention is not to be restricted except in light of the attached claims.

## Claims

1. A stent graft having a radially compressed configuration and a radially expanded configuration, comprising:
a tubular graft body (20) having a first end opening and a second end opening;
a stent structure (40) coupled along the graft body, wherein an end of the stent structure that is in closest proximity to the first end opening of the graft body is spaced from said first end opening to define a clearance region of the graft body; and
an annular mesh top stent (60) having a first portion (62) coupled along an inner surface of the graft body along the clearance region below the first end opening at attachment locations, and a second portion (68) extending beyond the first end opening outside of the graft body,
wherein, during radial expansion, the first portion (62) is retracted to a retracted longitudinal length, and the second portion expands to a bulbous shape having a greater cross-sectional area than the first portion, wherein the attachment locations are circumferentially spaced from one another at non-axially adjacent locations, whereby tearing of the graft body is inhibited during retraction of the annular mesh top stent.

2. The stent graft of claim 1, wherein, in a radially expanded configuration, a cross-sectional shape of the second portion (68) is a three-sided shape.

3. The stent graft of claim 1 or 2, wherein, in the radially expanded configuration, a cross-sectional shape of the first portion (62) has a rounded shape.

4. The stent graft of any preceding claim, wherein the stent structure (40) comprises a plurality of discrete stents coupled along the graft wall and axially spaced from one another.

5. The stent graft of claim 4, wherein each of the discrete stents is a self-expanding stent.

6. The stent graft of claim 4 or 5, wherein at least one of the discrete stents is disposed along an outer surface of the wall.

7. The stent graft of any preceding claim, wherein the annular mesh top stent (60) comprises a shape memory material.

8. The stent graft of any preceding claim, wherein the annular mesh top stent (60) is a self-expanding stent.

9. The stent graft of any preceding claim, wherein, in a radially compressed configuration, the second portion (68) has an extended length, and, in the radially expanded configuration, the second portion (68) has a retracted length less than the extended length of the second portion (68).

10. The stent graft of any preceding claim, wherein the annular mesh top stent (60) has a lower cell opening-to-stent material ratio than the stent structure.

11. The stent graft of any preceding claim, wherein a ratio between a diameter of the second portion (68) to a diameter of the first portion (62) is between 1.25 and 1.35.

12. The stent graft of any preceding claim, wherein the attachment locations are suture attachment locations arranged in a single row in an annular arrangement.

## Patentansprüche

1. Stentgraft mit einer radial komprimierten Konfiguration und einer radial expandierten Konfiguration, umfassend:
einen rohrförmigen Graftkörper (20) mit einer ersten Endöffnung und einer zweiten Endöffnung;
eine Stentstruktur (40), die entlang des Graftkörpers gekoppelt ist, wobei ein Ende der Stentstruktur, das sich in engster Nähe zu der ersten Endöffnung des Graftkörpers befindet, von der ersten Endöffnung beabstandet ist, um eine Freiraumregion des Graftkörpers zu definieren; und
einen kranzförmigen oberen Mesh-Stent (60) mit einem ersten Anteil (62), der entlang einer Innenfläche des Graftkörpers entlang der Freiraumregion unterhalb der ersten Endöffnung an Befestigungsstellen gekoppelt ist, und einem zweiten Anteil (68), der sich außerhalb des Graftkörpers über die erste Endöffnung hinaus erstreckt,
wobei der erste Anteil (62) während der radialen Expansion zu einer zurückgezogenen längsgerichteten Länge zurückgezogen wird, und der zweite Anteil zu einer Knollenform mit einer größeren Querschnittfläche als der erste Anteil expandiert, wobei die Befestigungsstellen an nicht-axial benachbarten Stellen im Umkreis voneinander beabstandet sind, während das Reißen des Graftkörpers während des Zurückziehens des kranzförmigen oberen Mesh-Stents verhindert wird.

2. Stentgraft nach Anspruch 1, wobei in einer radial expandierten Konfiguration eine Querschnittform des zweiten Anteils (68) eine dreiseitige Form ist.

3. Stentgraft nach Anspruch 1 oder 2, wobei in der radial expandierten Konfiguration eine Querschnittform des ersten Anteils (62) eine gerundete Form aufweist.

4. Stentgraft nach einem der vorhergehenden Ansprüche, wobei die Stentstruktur (40) eine Vielzahl diskreter Stents umfasst, die entlang der Graftwand gekoppelt und axial voneinander beabstandet sind.

5. Stentgraft nach Anspruch 4, wobei jeder der diskreten Stents ein selbstexpandierender Stent ist.

6. Stentgraft nach Anspruch 4 oder 5, wobei mindestens einer der diskreten Stents entlang einer Außenfläche der Wand angeordnet ist.

7. Stentgraft nach einem der vorhergehenden Ansprüche, wobei der kranzförmige obere Mesh-Stent (60) ein Formgedächtnismaterial umfasst.

8. Stentgraft nach einem der vorhergehenden Ansprüche, wobei der kranzförmige obere Mesh-Stent (60) ein selbstexpandierender Stent ist.

9. Stentgraft nach einem der vorhergehenden Ansprüche, wobei der zweite Anteil (68) in einer radial komprimierten Konfiguration eine ausgezogene Länge aufweist, und der zweite Anteil (68) in der radial expandierten Konfiguration eine zurückgezogene Länge aufweist, die kleiner als die ausgefahrene Länge des zweiten Anteils (68) ist.

10. Stentgraft nach einem der vorhergehenden Ansprüche, wobei der kranzförmige obere Mesh-Stent (60) ein niedrigeres Verhältnis von Zellöffnung-zu-Stentmaterial als die Stentstruktur aufweist.

11. Stentgraft nach einem der vorhergehenden Ansprüche, wobei ein Verhältnis von einem Durchmesser des zweiten Anteils (68) zu einem Durchmesser des ersten Anteils (62) zwischen 1,25 und 1,35 liegt.

12. Stentgraft nach einem der vorhergehenden Ansprüche, wobei die Befestigungsstellen Nahtbefestigungsstellen sind, die in einer einzelnen Reihe in einer kranzförmigen Anordnung angeordnet sind.

## Revendications

1. Greffon d'endoprothèse ayant une configuration comprimée radialement et une configuration expansée radialement, comprenant :
un corps de greffon tubulaire (20) ayant une première ouverture d'extrémité et une deuxième ouverture d'extrémité ;
une structure d'endoprothèse (40) accouplée le long du corps de greffon, une extrémité de la structure d'endoprothèse qui est le plus près de la première ouverture d'extrémité du corps de greffon étant espacée de ladite première ouverture d'extrémité pour définir une région de dégagement du corps de greffon ; et
une endoprothèse supérieure à maillage annulaire (60) ayant une première portion (62) accouplée le long d'une surface intérieure du corps de greffon le long de la région de dégagement en dessous de la première ouverture d'extrémité à des emplacements de fixation, et une deuxième portion (68) s'étendant au-delà de la première ouverture d'extrémité à l'extérieur du corps de greffon,
la première portion (62), au cours de l'expansion radiale, étant rétractée à une longueur longitudinale rétractée et la deuxième portion se déployant à une forme bulbeuse ayant une surface en section transversale supérieure à la première portion, les emplacements de fixation étant espacés circonférentiellement les uns des autres à des emplacements non axialement adjacents, le greffon d'endoprothèse ne pouvant pas se déchirer au cours de la rétraction de l'endoprothèse supérieure à maillage annulaire.

2. Greffon d'endoprothèse selon la revendication 1, dans lequel, dans une configuration radialement expansée, une forme en section transversale de la deuxième portion (68) est une forme à trois côtés.

3. Greffon d'endoprothèse selon la revendication 1 ou 2, dans lequel, dans la configuration radialement expansée, une forme en section transversale de la première portion (62) présente une forme arrondie.

4. Greffon d'endoprothèse selon l'une quelconque des revendications précédentes, dans lequel la structure d'endoprothèse (40) comprend une pluralité d'endoprothèses distinctes accouplées le long de la paroi de greffon et espacées axialement les unes des autres.

5. Greffon d'endoprothèse selon la revendication 4, dans lequel chacune des endoprothèses distinctes est une endoprothèse auto-expansible.

6. Greffon d'endoprothèse selon la revendication 4 ou 5, dans lequel au moins l'une des endoprothèses distinctes est disposée le long d'une surface extérieure de la paroi.

7. Greffon d'endoprothèse selon l'une quelconque des revendications précédentes, dans lequel l'endoprothèse supérieure à maillage annulaire (60) comprend un matériau à mémoire de forme.

8. Greffon d'endoprothèse selon l'une quelconque des revendications précédentes, dans lequel l'endoprothèse supérieure à maillage annulaire (60) est une endoprothèse auto-expansible.

9. Greffon d'endoprothèse selon l'une quelconque des revendications précédentes, dans lequel, dans une configuration comprimée radialement, la deuxième portion (68) présente une longueur prolongée et, dans la configuration expansée radialement, la deuxième portion (68) présente une longueur rétractée inférieure à la longueur prolongée de la deuxième portion (68).

10. Greffon d'endoprothèse selon l'une quelconque des revendications précédentes, dans lequel l'endoprothèse supérieure à maillage annulaire (60) présente un rapport d'ouverture de cellule à matériau de greffon d'endoprothèse inférieur à la structure d'endoprothèse.

11. Greffon d'endoprothèse selon l'une quelconque des revendications précédentes, dans lequel un rapport d'un diamètre de la deuxième portion (68) à un diamètre de la première portion (62) est compris entre 1,25 et 1,35.

12. Greffon d'endoprothèse selon l'une quelconque des revendications précédentes, dans lequel les emplacements de fixation sont des emplacements de fixation de sutures disposés dans une seule rangée suivant un agencement annulaire.
